# EUROPEAN PATENT APPLICATION

(11) **EP 1 306 432 A1**
(43) Date of publication of application: **02.05.2003**
(21) Application number: 01943855.5
(22) Date of filing: 28.06.2001
(51) Int. Cl.: C12N 15/11, C12N 5/10, C12Q 1/02, C12Q 1/68

(54) **HUMAN II TYPE 5 ALPHA-REDUCTASE PROMOTER GENE AND USE THEREOF**

(30) Priority: 28.07.2000 JP 2000228757
(71) Applicant: SHISEIDO COMPANY LIMITED, Chuo-ku, Tokyo 104-8010 (JP)
(72) Inventor: NAKANISHI, Jotaro, c/o Shiseido Research Center, Yokohama-shi, kanagawa 236-8643 (JP); HIBINO, Toshihiko, c/o Shiseido Research Center, Yokohama-shi, Kanagawa 236-8643 (JP)
(74) Representative: Hiebl, Inge Elisabeth
(86) International application number: JP0105549
(87) International publication number: WO02010376

(57) **Abstract**

A DNA molecule containing the promoter gene region of the human type II 5α-reductase gene, its modifications, and a method for screening type II 5α-reductase transcription controllers by using the same.

## Description

### Technical Field

This invention relates to DNA molecules which can act as a promoter for the human type II 5α-reductase gene and may optionally have an appropriate reporter gene operably linked thereto, as well as recombinant expression vectors thereof and *Escherichia coli* or mammalian cells containing such vectors. This invention also relates to uses of these DNA molecules and cells.

### Background Art

5α-Reductase is an enzyme catalyzing the 5α-position reduction of 4-ene-3-keto-steroids such as testosterone, 4-androstenedione and progesterone. Its typical role is to metabolize testosterone to 5α-dihydrotestosterone (DHT) that is the most powerful androgen. Although DHT performs important physiological functions (e.g., the differentiation of male external genital organs including the prostate gland at the embryonic stage and the manifestation of secondary sex characters at puberty) especially in males, it has been proved that DHT is involved in androgen-dependent diseases such as prostatomegaly and prostatic carcinoma, and dermatological diseases such as androgenetic alopecia, polytrichia, seborrheic dermatitis and acne vulgaris. In order to prevent or treat these diseases, suppression of DHT production in target cells is considered to be effective and various 5α-reductase inhibitors are being sought as a means therefor.

It has been clarified that there are two isozymes (i.e., type I and type II) of 5α-reductase, and their respective cDNAs have been cloned (Proc. Natl. Acad. Sci. USA 87:3640-3644, 1990, Nature 354:159-161, 1991).

In male adults, type I is relatively strongly expressed in the skin and the liver, while its weak activity is widely distributed over various parts of the body. On the other hand, type II is localized in androgen target tissues such as the prostate, epididymides and seminal vesicles (J. Clin. Invest. 92:903-910, 1993). The development of the prostate is controlled by DHT that is produced by 5α-reductase, and an elevation in 5α-reductase activity in prostatomegaly and prostatic carcinoma has also been reported (J. Clin. Endocrinol. Metab. 67:806-816, 1988). In recent years, various 5α-reductase inhibitors have been developed. Among others, finasteride that is an specific inhibitor of type II 5α-reductase is known to exhibit an excellent therapeutic effect on these diseases. Moreover, finasteride also exhibit a therapeutic effect on androgenetic alopecia and polytrichia (Biomed & Pharmacother 49:319-324, 1995).

As described above, type II 5α-reductase inhibitors are effective for certain androgen-dependent diseases, and some of them have been developed by evaluating an inhibitory activity against 5α-reductase. However, it would be desirable to provide a variety of drugs having a site of action different from that of 5α-reductase inhibitors.

### Disclosure of the Invention

In order to meet this demand, the present inventors have made investigations with a view to establishing an evaluation system which can control the *in vivo* production of type II 5α-reductase itself. As a result, it has been found that a polynucleotide of a particular region, in which the transcription starting point of the human type II 5α-reductase gene has been elucidated (Endocrinology 131:1571-1573, 1992) but a region including the transcription starting point has not been fully analyzed as yet, and its certain modifications participate closely in the transcriptional control of the type II 5α-reductase gene.

The present invention has been completed on the basis of these findings.

Accordingly, one embodiment of the present invention relates to an isolated DNA molecule capable of acting as a promoter for the type II 5α-reductase gene derived from human beings. This DNA molecule is selected from the group consisting of:
(a) a polynucleotide comprising the DNA sequence represented by SEQ ID NO:1, the polynucleotide comprising a continuous DNA sequence extending from position 1 to 6022;
(b) a fragment of polynucleotide (a), the fragment comprising a continuous DNA sequence of at least about 50 nucleotides including the transcription starting point at position 5952 (T); and
(c) a polynucleotide having the DNA sequence of polynucleotide (a) in which one or more nucleotides have been modified by substitution, deletion and/or addition, and containing a polynucleotide comprising a continuous DNA sequence of at least about 50 nucleotides including the transcription starting point at position 5952 (T).

The present invention also relates to uses of this DNA molecule, including a recombinant expression vector carrying the DNA molecule, and *Escherichia coli* or a mammalian cell containing the DNA molecule or the recombinant expression vector. The DNA molecule may further contain a reporter gene operably linked thereto. When the DNA molecule further contains a reporter gene, there can be provided a system for evaluating the human type II 5α-reductase transcription controlling ability of various test samples by using a mammalian cell as described above. Such test samples may be any compounds including natural organic compounds and chemically synthesized compounds. This evaluation system may be applied, for example, to a method for screening drugs having a human type II 5α-reductase transcription controlling ability.

Thus, the present invention not only provides a means for elucidating the mechanism controlling the expression of the human type II 5α-reductase gene, but also serves to diagnose, prevent or treat diseases associated with the expression thereof. Moreover, there is a possibility that the present invention may be utilized, for example, for the purpose of screening drugs which are highly effective in promoting the production of type II 5α-reductase (or activating the aforesaid transcription) or suppressing the production of type II 5α-reductase (or inactivating the aforesaid transcription) in various types of cells.

### Brief Description of the Drawings

FIG. 1 illustrates the structure of the 5'-upstream region of the human type II 5α-reductase gene represented by SEQ ID NO:1, in which the probe was used for the screening of a human genome library; and
FIG. 2 is a photograph showing the results of an SRY over-expression experiment with cultured human dermal papilla cells, as described in Example 5.

### Best Mode for Carrying Out the Invention

The term "promoter" as used herein means a region of DNA which is involved in the efficiency of the transcription initiation reaction. Accordingly, the expression "capable of acting as an operator" is used interchangeably with "having a transcription controlling ability", and ultimately means that the DNA molecule has a transcription activating or inactivating effect which controls the expression of human type II 5α-reductase positively or negatively, respectively. In the present invention, greater emphasis is placed on, but is not limited to, a transcription inactivating effect.

First of all, the expression "operably linked" as used herein means that an objective polynucleotide (e.g., a reporter gene) attached to the promoter or a transcription control region (or a region having a transcription controlling ability) is linked in such a manner as to be expressible in certain host cells. Generally, the polynucleotide is linked on the downstream side of a transcription control region. In this case, a polynucleotide (or oligonucleotide) having no adverse influence on the expression of the objective polynucleotide may intervene between the transcription control region and the objective polynucleotide.

One isolated DNA molecule in accordance with the present invention is a polynucleotide comprising the DNA sequence represented by SEQ ID NO:1, the polynucleotide comprising a continuous DNA sequence extending from position 1 to 6022. For example, this polynucleotide may be prepared in the following manner. First of all, a DNA fragment comprising a region in the neighborhood of the transcription starting point of human type II 5α-reductase is obtained by a *per se* known PCR process using primers represented by SEQ ID NO:2 and 3 and human genomic DNA. This DNA fragment is labeled with digoxigenin and used as a probe for the screening of a human genomic DNA library. Then, phage DNA is prepared from positive plaques and digested with appropriate restriction enzymes. Thus, a desired DNA fragment is identified by Southern blotting using the aforesaid probe. This DNA fragment is subcloned into pBluescript II vector.

Another isolated DNA molecule in accordance with the present invention is a fragment of the aforesaid polynucleotide, the fragment comprising a continuous DNA sequence of at least about 50 nucleotides including the transcription starting point at position 5952 (T) in the sequence of SEQ ID NO:1. Moreover, such fragments must be able to act as a promoter for the type II 5α-reductase gene derived from human beings. These fragments may be any DNA molecules that satisfy the above-described requirements. The preparation of these fragments may be carried out according to the procedure described in Example 2 which will be given later. Specifically, these fragments may be obtained by digesting a polynucleotide comprising the DNA sequence represented by SEQ ID NO:1 with appropriate restriction enzymes in such a way as to cause the aforesaid transcription starting point to remain.

A further isolated DNA molecule in accordance with the present invention is a polynucleotide having the aforesaid basic DNA sequence (i.e., the continuous DNA sequence extending from position 1 to 6022 in which one or more nucleotides have been modified by substitution, deletion and/or addition, and containing a polynucleotide comprising a continuous DNA sequence of at least about 50 nucleotides including the transcription starting point at position 5952 (T). Moreover, such polynucleotides must also be able to act as a promoter for the type II 5α-reductase gene derived from human beings. Furthermore, polynucleotides modified by deletion include polynucleotides preferably comprising a sequence in which one or more nucleotides are deleted so that any duplication of the aforesaid fragments can be avoided and the basic DNA sequence will be interrupted. The site(s) of deletion may be arbitrary in position and number, provided that the above-described requirements are satisfied, i.e., the transcription starting point remains and the polynucleotide can act as a promoter. However, it is usually preferable that they are positioned so as to cause one or more of the plurality of transcription factor binding motifs (as will be described later) to lose their functions (i.e., the corresponding transcription factors come to be unable to bind to them). Moreover, with respect to substitution, one or more bases (A, T, C, G) in the basic DNA sequence may be replaced by any other bases, provided that the above-described requirements are satisfied. Alternatively, two or more consecutive bases may be replaced. Such substitution may also be effected so that some of the aforesaid transcription factor binding motifs will lose their functions. Furthermore, addition comprehends the addition of a nucleotide or a poly- or oligonucleotide to the 5'- or 3'-terminus, and the addition of the aforesaid nucleotide or the like so as to force itself into the basic DNA sequence. Of course, it is required to satisfy the above-described requirements.

Two or more of these substitution, deletion and addition may occur concurrently. They may be effected by *per se* known techniques such as digestion with restriction enzymes and ligation with ligase, the introduction of a site-specific mutation, and PCR.

The isolated DNA molecule as described above may further contain a reporter gene operably linked thereto. Usually, the reporter gene is linked on the downstream side of the DNA molecule so as to cause the frames to match with each other, optionally through the medium of a nucleotide sequence which has no adverse influence on the expression of the gene. Although the reporter gene may be any of those known in the art, it is preferable to use a reporter gene whose expression can be easily detected. Such reporter genes include, but are not limited to, the luciferase gene and the β-galactosidase gene. As the aforesaid linking method, there may be used a *per se* known method.

The present invention also provides vectors, preferably expression vectors, carrying the above-described DNA molecule or a structure comprising the DNA molecule containing a reporter gene operably linked thereto. One type of such vectors may be plasmids, and another type thereof may be vectors derived from viruses. These vectors can autonomously replicate in host cells into which they have been introduced. In this description, such vectors are referred to as expression vectors or recombinant expression vectors.

For the construction of such expression vectors, a wide variety of vectors may be used according to the type of host cells to be transfected. For the purposes of the present invention, however, it is convenient to use, for example, pBluescript II (manufactured by Stratagene) when *Escherichia coli* can be used as the host, and pGL 3 basic vector (manufactured by Promega) when a mammalian cell is used as the host. The construction of such expression vectors may be carried out according to a *per se* known method.

The present invention further provides *Escherichia coli* or a mammalian cell containing the aforesaid expression vector or recombinant expression vector, or the aforesaid DNA molecule which may have a reporter gene operably linked thereto. The introduction or transfection of an exogenous DNA molecule comprising the expression vector or the DNA molecule into host cells (*Escherichia coli* or mammalian cells) may also be carried out according to a *per se* known method. Such methods include coprecipitation with calcium phosphate or calcium chloride, DEAE-dextran-mediated transfection, lipofection and electroporation. Methods suitable for the transformation or transfection of host cells can be found in Sambrook et al., "Molecular Cloning: A Laboratory Manual" (Second Edition, Cold Spring Harbor Laboratory, Cold Spring Harbor Laboratory Press, Cold Spring Harbor, NY, 1989) and other laboratory manuals.

The *Escherichia coli* into which the aforesaid exogenous DNA molecule has been introduced (e.g., JM 109 strain) serves to amplify the DNA molecule, while the mammalian cell into which the aforesaid exogenous DNA molecule has been introduced may be utilized in a method for evaluating the human type II 5α-reductase transcription controlling ability of test samples, as will be described later. The mammalian cells which can be used to accomplish this purpose include human prostatic stroma cells, human foreskin fibroblasts, human dermal papilla cells and the like.

The aforesaid evaluation method may be carried out by introducing the aforesaid DNA molecule (in particular, the one containing a reporter gene) into a mammalian cell, culturing the mammalian cell in the presence of a test sample, and using a change in the degree of expression of the reporter gene in the culture as an indicator of the human type II 5α-reductase transcription controlling ability of the test sample. The degree of expression of the reporter gene can be determined by measuring the level of the expression product by an appropriate detection means selected according to the reporter gene used, and a change in the degree of expression can be determined, for example, by comparing the measured degree of expression with the degree of expression of the reporter gene in a culture (control) obtained by culturing the cell under the same conditions, except that the test sample is absent. The cell may be cultured under any conditions known in the art, depending on the type of the host cell used.

The above-described evaluation method may be applied to a method for screening type II 5α-reductase controllers from compound libraries or natural substances according to a high throughput screening technique (Nature, 384, supp., 14-16, 1996) or the like. Specifically, the cell is treated with a test compound for a suitable period of time and its reporter activity is measured. Thus, substances capable of raising or reducing the activity are screened. The drugs obtained in this manner will be able to control the expression of human type II 5α-reductase, either directly by acting, for example, on cis-elements or transcription factors, or indirectly by controlling the signal transfer system.

Moreover, with respect to the DNA sequence represented by SEQ ID NO:1, transcription factors participating in the transcriptional control of type II 5α-reductase can be predicted by retrieving transcription factor binding motifs on the basis of a database such as TRANSFAC. In practice, when retrieval was carried out with respect to the entire DNA sequence represented by SEQ ID NO:1, the presence of a large number of transcription factor binding motifs including SP-1, AP-1, CREBP1, Nkx-2.5, SOX5 and the like was recognized. It is believed that these transcription factors act on cis-elements in the type II 5α-reductase transcription control region and thereby promote or suppress the transcription of type II 5α-reductase. Accordingly, substances capable of controlling the production of these transcription factors, or substances inhibiting the binding of transcription factors to cis-elements will also be useful as type II 5α-reductase transcription controllers.

An actual example in which these transcription factors control the expression of type II 5α-reductase is the promotion of the expression of type II 5α-reductase by SRY (sex determining region Y). Specific results thereof are shown in Example 5. Accordingly, substances having a site of action involving the inhibition of SRY production or the inhibition of the binding of SRY to a cis-element will be useful as type II 5α-reductase transcription suppressor.

Accordingly, the present invention also provides a method for promoting or suppressing the expression of human type II 5α-reductase which comprises providing an expression vector containing an isolated DNA molecule encoding transcription factors for transcription factor binding motifs present in the DNA sequence represented by SEQ ID NO:1, and culturing a mammalian cell having the expression vector introduced thereinto; and a method for evaluating the human type II 5α-reductase transcription controlling ability of a test sample which comprises carrying out the aforesaid method in the presence of a test sample and using a change in the amount of expression of human type II 5α-reductase caused by the presence of the test sample, as an indicator of the human type II 5α-reductase transcription controlling ability of the test sample. This evaluation method can in turn be applied to a method for the screening of type II 5α-reductase transcription controllers. The mammalian cells which can be used in the aforesaid evaluation method by introducing thereinto an expression vector containing a DNA molecule encoding transcription factors for transcription factor binding motifs include, for example, human prostatic stroma cells, human foreskin fibroblasts and human dermal papilla cells which may contain the aforesaid DNA molecule having a reporter gene operably linked thereto as an exogenous DNA molecule.

Type II 5α-reductase transcription suppressors which can be obtained by the screening method of the present invention will be effective for the prevention and treatment of androgen-dependent diseases such as prostatomegaly and prostatic carcinoma, or androgenetic alopecia and the like.

Now, the present invention is more specifically explained with reference to the following examples. However, these examples are not to be construed to limit the scope of the invention.

### Example 1: Cloning of genomic DNA of human type II 5α-reductase

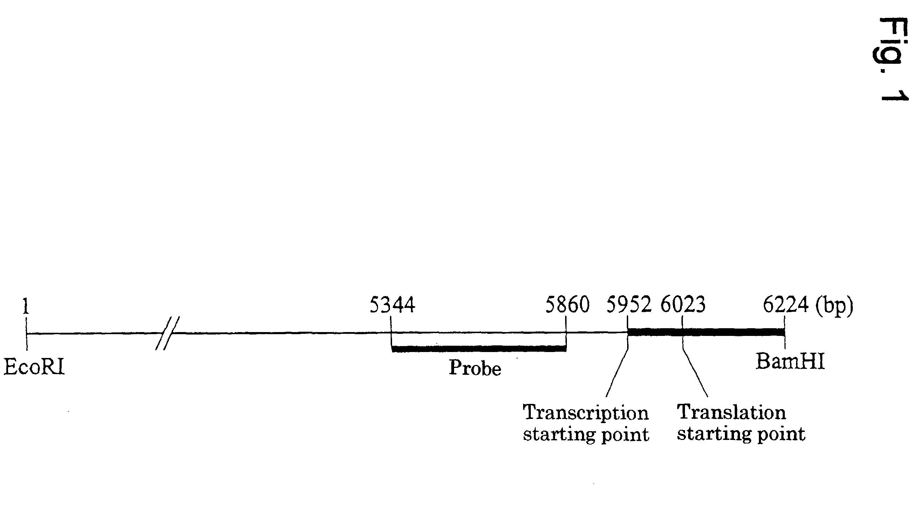

A DNA fragment of a region in the neighborhood of the transcription starting point of human type II 5α-reductase, which is represented by SEQ ID NO:4, was obtained by PCR using primers represented by SEQ ID NO:2 and 3 and genomic DNA of human placenta. gaaaccgctg aggaattagg (SEQ ID NO:2) ttcgcagcaa taccccctttc (SEQ ID NO:3) This DNA fragment was labeled with digoxigenin and used as a probe to screen a human genomic DNA library. Specifically, *Escherichia coli* K802 strain was infected with a human genomic DNA library (vector: EMBL3 SP6/T7) manufactured by Clontech, and allowed to form plaques. After these plaques were transferred to a nylon membrane, hybridization was carried out by using the aforesaid probe. For the purpose of detection, a color reaction was carried out by using alkaline phosphatase-labeled anti-digoxigenin antibody (manufactured by Roche Diagnostics). As a result of the screening of about 2,000,000 plaques, two positive plaques #1 and #2 were obtained. Phage DNA was prepared therefrom, digested with various restriction enzymes, and then subjected to Southern blotting using the aforesaid probe. As a result, a plurality of DNA fragments proved to be positive. It was confirmed that, on the basis of the known base sequence and restriction map of the human type II 5α-reductase gene, a DNA fragment of about 8 kb obtained by digesting phage DNA #1 with BamHI covered the 5'-upstream region including the transcription starting point over the longest range. Consequently, this DNA fragment was used for subsequent experiments. A fragment of about 6.2 kb obtained by digesting this DNA fragment with EcoRI was subcloned into pBluescript II vector which had been similarly digested with BamHI and EcoRI. This plasmid was named p6.2BS. The entire base sequence of the inserted fragment of about 6.2 kb is shown as SEQ ID NO:1, and its structure is shown in FIG. 1.

### Example 2: Construction of a reporter plasmid comprising the human type II 5α-reductase promoter and the luciferase reporter gene

The DNA fragment represented by SEQ ID NO:1 contains, in addition to the 5'-upstream region of the human type II 5α-reductase gene, a portion of the structural gene following the translation stating codon (ATG) (from position 6023 to 6224 in SEQ ID NO:1). Accordingly, if the luciferase gene is directly liked thereto, the expression of luciferase cannot be expected owing to a frame shift. For this reason, the procedure for removing the structural gene portion of type II 5α-reductase from p6.2BS plasmid was carried out as follows. First of all, p6.2BS plasmid was digested with SacII to remove a portion extending from the SacII site at position 5823 in SEQ ID NO:1 to the SacII site derived from pBluescript II. On the other hand, using PCR primers having a SacII recognition sequence attached to the 5'-terminus and represented by SEQ ID NO:5 and 6, PCR was carried out by using p6.2BS plasmid as a template. Thus, there was obtained a DNA fragment extending from base "C" at position 5820 in SEQ ID NO:1 (3 bases before the SacII site) to base "G" at position 6022 immediately before the translation starting point. This fragment was digested with SacII and then ligated into p6.2BS plasmid which had been digested with SacII as described above. The direction of the inserted fragment was determined, and a clone having the same direction as SEQ ID NO:1 was selected. This plasmid was named p6.0BS. Then, p6.0BS plasmid was digested with BssHII and the resulting about 6 kb DNA fragment of the human type II 5α-reductase transcription control region was subcloned into pGL3 basic vector which had been digested with KpnI and HindIII. The plasmid thus obtained was named pRedII-Luc. Furthermore, pRedII-Luc was digested with a restriction enzyme to remove the 5' side from the cleavage site in the inserted type II 5α-reductase transcription control region DNA. Thereafter, this plasmid was self-ligated to prepare a modification of pRedII-Luc. The names of actually prepared plasmid modifications and the restriction enzymes used for their preparation are shown in Table 1.

**Table 1**

| Name of plasmid | Restriction enzymes |
|---|---|
| pRedII/ApaI-Luc | ApaI |
| pRedII/SnaBI-Luc | SnaBI, XhoI |
| pRedII/PstI-Luc | PstI, XhoI |
| pRedII/BalI-Luc | BalI, XhoI |
| pRedII/NsiI-Luc | NsiI, XhoI |
| pRedII/BstXI-Luc | BstXI, XhoI |
| pRedII/HindIII-Luc | HindIII, XhoI |
| pRedII/PflMI-Luc | PflMI, XhoI |
| pRedII/KpnI-Luc | KpnI |
| pRedII/Eco065I-Luc | Eco065I, XhoI |
| pRedII/StuI-Luc | StuI, XhoI |

### Example 3: Measurement of promoter activities for human type II 5α-reductase

In this example, each of the aforesaid reporter plasmids was transfected into human dermal papilla cells and its promoter activity was measured.

On the day before transfection, a 24-well plate was inoculated with an equal number of cells. On the next day, each plasmid and pSV-β-Galactosidase Control Vector (manufactured by Promega), containing the β-galactosidase gene as an internal control for the measurement of gene introduction efficiency, were mixed with Lipofectamine Plus Reagent (manufactured by Lifetech Oriental), and this mixture was added to the cells in order to introduce the genes thereinto. After being incubated for 24 to 48 hours, the cells were lysed and measured for luciferase activity and β-galactosidase activity. The luciferase activity was measured by means of a Picagene Luminescence Kit (manufactured by Toyo Ink), and the β-galactosidase activity was measured by using CPRG as a color-producing substrate. The results thus obtained are shown in Table 2. The promoter activity was obtained by correcting the luciferase activity for the β-galactosidase activity and expressing it as a relative value based on the promoter activity of pRedII-Luc. Moreover, it has been confirmed that a DNA fragment of up to about -60 bp, inclusive of the transcription starting point, also has a strong promoter activity.

**Table 2**

| Name of plasmid | Size from transcription starting point to 5'-terminus of transcription control region (bp) | Promoter activity |
|---|---|---|
| pRedII-Luc | 5952 | 100 |
| pRedII/ApaI-Luc | 4763 | 587 |
| pRedII/SnaBI-Luc | 3553 | 393 |
| pRedII/PstI-Luc | 2949 | 1380 |
| pRedII/BalI-Luc | 2370 | 1512 |
| pRedII/NsiI-Luc | 2093 | 1586 |
| pRedII/BstXI-Luc | 1783 | 1385 |
| pRedII/HindIII-Luc | 1576 | 1652 |
| pRedII/PfIMI-Luc | 837 | 1943 |
| pRedII/KpnI-Luc | 570 | 2362 |
| pRedII/Eco065I-Luc | 346 | 2763 |
| pRedII/StuI-Luc | 149 | 2822 |
| pGL3 basic | - | 0 |

### Example 4: Screening of type II 5α-reductase transcription controllers

pRedII-Luc and pSV-β-Galactosidase Control Vector are transfected into dermal papilla cells in the same manner as in Example 3. After 24 hours, various substances are added and the incubation is continued for an additional 24-48 hours. Thereafter, the promoter activity is measured in the same manner as in Example 3. Thus, various substances capable of promoting or suppressing the expression of type II 5α-reductase can be screened.

### Example 5: Promotion of the transcription of type II 5α-reductase by transcription factor SRY (sex determining region Y)

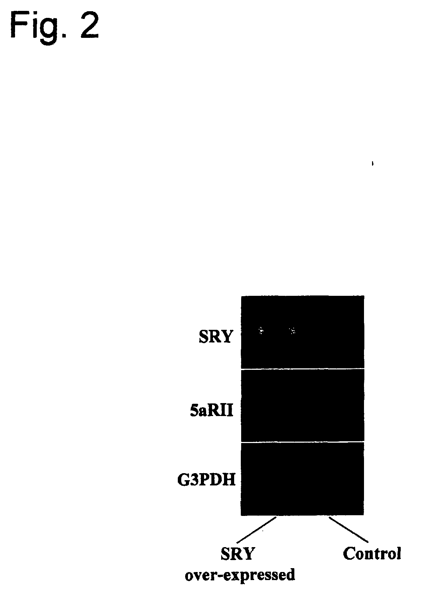

With respect to the base sequence of the type II 5α-reductase transcription control region DNA, transcription factor binding motifs were retrieved on the basis of TRANSFAC database. As a result, a biding motif for the transcription factor SRY was recognized. This suggests that the transcription of type II 5α-reductase may be controlled by SRY. Then, a change in the expression of type II 5α-reductase due to an over-expression of SRY was examined by using dermal papilla cells. Specifically, using PCR primers having an EcoRI recognition sequence attached to the 5'-terminus and represented by SEQ ID NO:7 and 8, the full-length structural gene of SRY was obtained by using human genomic DNA as a template. This fragment was digested with EcoRI and then subcloned into the expression vector pVP22/myc-His (manufactured by Invitrogen) which had been similarly digested with EcoRI. This plasmid was named pVP22-SRY/myc-His. pVP22-SRY/myc-His and pVP22/myc-His as a negative control were separately transfected into hair papillary cells in the same manner as in Example 3. After being incubated for 48 hours, the cells were harvested and RNA was extracted therefrom. Then, the expression of mRNAs for SRY and type II 5α-reductase (5aRII) was examined by RT-PCR. The results thus obtained are shown in FIG. 2. Owing to the introduction of pVP22-SRY/myc-His, the amount of expression of SRY was increased about tenfold and that of type II 5α-reductase was increased about fourfold. These results have revealed that SRY promotes the expression of type II 5α-reductase.

### Industrial Applicability

According to the present invention, substances capable of controlling the expression of human type II 5α-reductase positively or negatively can be screened by providing a recombinant expression vector comprising a 5'-upstream gene containing a promoter for human type II 5α-reductase and an appropriate reporter gene linked thereto, introducing this vector into an animal cell, and using the resulting reporter activity as an indicator. In particular, substances capable of suppressing the expression of the human type II 5α-reductase gene will be effective for the prevention and treatment of androgen-dependent diseases such as prostatomegaly and prostatic carcinoma, or male pattern alopecia and the like. Accordingly, the present invention can be utilized in the pharmaceutical industry, the cosmetic industry and the like.

## Claims

1. An isolated DNA molecule capable of acting as a promoter for the type II 5α-reductase gene derived from human beings, the DNA molecule being selected from the group consisting of:
(a) a polynucleotide comprising the DNA sequence represented by SEQ ID NO:1, the polynucleotide comprising a continuous DNA sequence extending from position 1 to 6022;
(b) a fragment of polynucleotide (a), the fragment comprising a continuous DNA sequence of at least about 50 nucleotides including the transcription starting point at position 5952 (T); and
(c) a polynucleotide having the DNA sequence of polynucleotide
(a) in which one or more nucleotides have been modified by substitution, deletion and/or addition, and containing a polynucleotide comprising a continuous DNA sequence of at least about 50 nucleotides including the transcription starting point at position 5952 (T).

2. A recombinant expression vector carrying the DNA molecule of claim 1.

3. An expression vector as claimed in claim 2 which further carries a reporter gene operably linked to the DNA molecule,

4. *Escherichia coli* or a mammalian cell containing the DNA molecule of claim 1 or the expression vector of claim 2.

5. *Escherichia coli* or a mammalian cell as claimed in claim 4 which further contains a reporter gene operably linked to the DNA molecule.

6. A method for evaluating the human type II 5α-reductase transcription controlling ability of a test sample which comprises culturing the mammalian cell of claim 5 in the presence of a test sample, and using a change in the degree of expression of the reporter gene in the culture as an indicator of the human type II 5α-reductase transcription controlling ability of the test sample.

7. A method for promoting or suppressing the expression of human type II 5α-reductase which comprises providing an expression vector containing an isolated DNA molecule encoding transcription factors for transcription factor binding motifs present in the DNA sequence represented by SEQ ID NO:1, and culturing a mammalian cell having the expression vector introduced thereinto.

8. A method for evaluating the human type II 5α-reductase transcription controlling ability of a test sample which comprises carrying out the method of claim 7 in the presence of a test sample, and using a change in the amount of expression of human type II 5α-reductase caused by the presence of the test sample, as an indicator of the human type II 5α-reductase transcription controlling ability of the test sample.


SEQUENCE LISTING

<110> SHISEIDO COMPANY, LTD.

<120> HUMAN II TYPE 5α-REDUCTASE PROMOTER GENE AND USE
THEREOF

<130> 13117EP

<140> EP 01 943 855.5
<141> 2001-06-28

<150> JP 2000/228757
<151> 2000-07-28

<160> 8

<210> 1
<211> 6224
<212> DNA
<213> Homo sapiens

<400> 1
gaattctctg tctgaatggt cctatgtctt gtttctccag gattggtcca tggtgcctta 60
cttagttcat ttggtgaagt catgttttcc tggattatct tgatacttgt aaatgttctt 120
tggtgttagg atttattgta gtcttcactg tctgtgtttt tttgttcctg tcctccttgg 180
gatggatttt cagatatttg aaaagaccgg agtattttga tctatgctgt atctgcttta 240
gagggtacca caatcccagt aatgctatgg ttcttgcagt ttctagatgt actgctttga 300
tggtcttgga caagatccag gataattctc tggattacca ggcagatact cttattttct 360
ttccttactt tctcccaaac agagtccctc tgtcagttct gagacacctg aagctaggag 420
tggagtgaaa ccagcacccc ggaggacacc accactatga ctgtgctcag ccagacttga 480
agccagcaca acactgggtc ttccctgctg taaccactcc ctggccactg cctatgtttg 540
ctcaaggcct tggggctcta caataaacag gtggaaaagc catccaggtc tgtgcccttc 600
ccttcagggt ggcaagttcc cccaagcccc aggtgggtcc agagatgttg tctaggagtc 660
agggactaga gtaaaaaacc tttgaagtct acctggcatt ctattgtatt aaaattgagc 720
tgggggggac tcgggggaaa gtgtgggagg ggagtagggg ataaaagact acacattggg 780
tacggcgtac accgcttggg tgatgggcac caaaatctca gaaatcacca ctaagaactt 840
atctgtgtaa ccaaacacca cctgttcccc aaaaacctat tgaaataata ataataataa 900
ataataataa aatgaataaa caaaaacact gagctggcac tcaaaccaca aaacacagtc 960

tttcccactc tttcctcccc tgtccaaagg cagaggagcc tcactccaca gcccacaaga 1020
agtactgctg gattatcact ggtattcatt taaggcagaa aggctattaa gtcagctggt 1080
ggtgactgct gcctggcctg ggactcactc tttggggcag tgggcttccc tgttggccca 1140
ggaaaggtct agaaatgctg ttgaagagtc aactcttgga atcaagggcc cccaagagct 1200
tgcttggtgc tttacctccc tgtggctgag ccagtacctg aaaccagcta gtctcagagg 1260
ctcacccaag gcccttgatg tagtatctgg gtatcactgc tggttattca gagccaaagg 1320
gcttttcagc tagcaggtga tgaacgctgc catgactggg tctttccctt caaagcagtg 1380
ggttcccttc tggcccaggg tgtttctagg aatgtcacct ggaagctagg gcctggaaca 1440
gggacctcat tattctgact ggtgccatat ctgttgtggc tgagctggta tccaagatgc 1500
aaggcagagt ctcctcaact ctttcctctc ctcttctcaa gcagcaggaa ggggtctctt 1560
ttagagttgt gagttgtgca gcctgggatt ggggaggggt gatgccagca ctcccttggc 1620
tgccccagct gggtgtctca gtatattttg cacccctcag tccactgtct ctggccctag 1680
ttcagtacta ggacttgtct aagaattgca gttcttttgg cctaaactgc ctttcaggtt 1740
tagtcagaga tccagagcac ttcagccctc agtggtgagg tttgtgggaa ctgaaattct 1800
gactgctgga attagtgatt cccctttggc tgggactggt ttgaatgctc cctttatgtg 1860
tgggcatcag ctgaactttg tctggctttc ctttttgctg taacaggaca acactgagtt 1920
taatgcctca ccattgatgt gttctccctc acccagtgca cgaaaatgct ctttgcacca 1980
caccacagct gccagggtgt gatggagggg tggcttcagt gcttcaagac tccttctgca 2040
accttttcag tgcctttttc agcaacacaa agttaaaagc aggtactgca agtgctcact 2100
tgagttttgg ttcttgtgaa ggagctttgc ttgcacagat agttgttaaa ttggtgtcct 2160
tgttggggaa acaatcagtg gagccttcta ttccaccatc ttgctccacc tcccatagta 2220
ctatacatct cttcttttgg ctagttctgt ttggatattt ttgctataat aaaactgtag 2280
ttgtaagtat agtgctttcc agagttcttt aatttttttt agtgaattat caagcttgaa 2340
gggttagtgg ggattcctaa atatggtagc cagctgttta gaagtatagg tagcttacgt 2400
aaccttgaac ttgcagctga tgtctgaagt aaggacagtg ttatggagga ccatgtcctt 2460
aacttttgaa gtttggctca actctagtag ttgttgtcaa aagttgatgt aacatgccta 2520
gtaattttag gaatgctgga tattgtaaag gctatattct tacttgtctg gatttaattg 2580
acttccttta aacagtgttg gactgtgtta ggttacttgt gagtcatctt gatcctttgg 2640
ggggattgat tttatgtttt gttaagaaga tagcttttat tctacctaat tccaatactc 2700
tgtagatcat ttctactact aaaccatggg cttctgggat ctcccagcta gctgtttcct 2760
ggagattata aatgactcta acacatcttt catatgcaaa ccaactaatt cagggctcat 2820
acgttcccca accacttcct ttatcaggac tctacaccct gggccactat tctcctgccc 2880
taatcagcca ggtccaggta acagaaaagt aaagacagcc gctgtacccc agagcctgct 2940
aaaagtattc aaacgagcta atcctaagcc tgattacctt gtcatgccca ctctttcctg 3000
cagaaactac agtaaaggct cttgcccacc ttgacccctc actccggctg cctcctaaca 3060

ctggtgcttc tccatgtggg cttgggtggt gtgctgtgtc ttctgtttgt agggatctgt 3120
cgatataaac cttttccttc acgatagtca tttctgtgtc tgcatatgtt accacattga 3180
ttaaaaagag taagtactgt atgattccac ttacccgaaa ttcaaaagca gacaaaatta 3240
acttatggtg ttagaagtca gggtagtggt gttttttttt tttgcttttt tttttttttt 3300
ttttttcctg gaaggaggat gtaaggaggc aggagcacag atcctgatat gctgataatc 3360
tgtatcttga cctaggtgca gcttgcaaga gggtgttcag tttgtgcaaa tcaaccaagc 3420
tgtatattta taattgggca ctttcttctt gcatgtaata cagaccagtt gcattattgg 3480
ttccaatttt ctacctccca ctatatccct gccctttacc atgtaacttt acaaatagtc 3540
tctcactttg gctctagaat ccaccatatg acttgatttg gccaatacaa taaataggaa 3600
gtaagggtgt gcttgttctt ggcctgagcc tgaagaggcc ttgtgtgttt cttctactct 3660
cgtacttttg ttattaacat tatatagaca tggtggagct agctcactca tcccaggatg 3720
agagcctcag ctaagtttct ccatccaaac acagcctgga gctgggctct aacttgttac 3780
acagatgaat gagtgaatat tgttaagata agtcaagccc agcccagatt tctgacccac 3840
agccaaccca cagatgcata agctgaagat gactggtttt atcaagctaa ttgttataat 3900
agtggagaaa agatcatgag gacaaaaagt gggcagagtc ggaagaaaag agaggaagaa 3960
attgagacag aagacatttc atttaaaaaa aatattccat tgagctgggt ttgaaatagt 4020
gcactgcctg ttctcctaat gctgtatggt gtcatgaaat ctattgttta ctgagtctat 4080
gagccagctt cctagggagg ctatggcaat tgaggacagg gaagaggtaa cactcaggaa 4140
catagaagga gaatttgggt ccaagtgggt ggagggaaaa ataactgggt ttagttttgg 4200
gtagggctgg ttttgaggtc tctgaaggac atgtaagtgg agttttccag cagggagaaa 4260
acgcagagct aaagctcata tctttgctgg caatctagat ttgggcatct tcaacaagca 4320
ggttgtagct gaggaagctg ggactggact tgtactctaa agccagtgca aaaaaagctt 4380
gaaacggcta tgatggctaa gacctggctt ttccatgaaa aatgcttcgg tcagtatgag 4440
tgattccaaa gtggtgatca attaaaaact gaagtatgat tagcattaat tataacccaa 4500
tgggaatatg ataaacgact cttggtcagc aagcagaggg tgccctgtag tgctaaagca 4560
tcaccatata ccatgtgtgc caagaatcag gagacacccc aaacgggagc agatgagggg 4620
ttgtgtctgt cattggacca gctggcctga tccagcagaa gtggatggag atacactgaa 4680
tggggctcct gggaggcgtg agttgaaggg gaaggaagag aagagacctc caaagtaagg 4740
gaagagtgaa aaatgagaag gactggggtg gagccccaag tagggaccag aggagaaaac 4800
agggataaag taatcaaggg agatggacaa ggaagatgaa agaatgaggt aaacagcagg 4860
tgggaagagg aggtcaacct aaaggagaaa gccgggtcga agaaagaagg aagagaagaa 4920
aagaagggtt gggaaacaga ggaggaggca gccaagaaag cctggaagct gaatcataga 4980
acggaagagg tagaagacgg aggggctgga ggataacata aaggtgggaa acggaagaga 5040
gaaagaaccg cgtctgcgtg tatgacggct agacaggagt tcagagaaca gcggggtcgc 5100
caggccacca cctgatgggc cacggctcat tggctctagg agctgggaaa gggcatccca 5160

ggaaagaagc cctagacttt agcctgagtc tgggccactc taggggaccg ggagtggggt 5220
ggcgggagag gacgcgcaga atctcgactt ctggccccaa tctgtgcatg atcacccgag 5280
ctcagcggac gctcctctct gacccaggca ggcggctcag ggacgcgtgc ggggatgcag 5340
agagaaaccg ctgaggaatt agggccggga gagactggta cctgccgggg gcgtgtggtg 5400
gggcagagct ggcactgatg ctgagagtgg ctaaggagcg cggcgcccca gagcagaagg 5460
gctggcagac gctcagagag ccaggatggt tcagggtcca aggaaggtcc tatgttgggt 5520
gggagctgtg agggagtgaa agtgcatgag gaaccggagg agatggaaag accttggctt 5580
gggtgttcga gggtgggact gcgtggtgac cgacggcaca gagggtgtgt gttggggcgg 5640
aagaaccacc ccagctgaat cgtccccgtg gggttttctt cccgtgtctt agttccagaa 5700
gttgccgcat cagacgctaa tagttgagga acaagtcatg gaaggacagc ctaagcggga 5760
ggtgaatgta aagccgtgga gagggcgggc gaactaagaa ggccttcgtt ctcctccggc 5820
caccgcggct gcatccttga gaaaggggta ttgctgcgaa gccgcgccag ggctggacgc 5880
ggcgaggtgg gaggcaggat ggaggggcgg gagccaaggc cgagggggcg gacacgggtg 5940
gcgtctggcg ctccataaag cggttgcggg ggccgcgctc tcttctggga gggcagcggc 6000
caccggcgag gaacacggcg cg atg cag gtt cag tgc cag cag agc cca gtg 6052
Met Gln Val Gln Cys Gln Gln Ser Pro Val
1               5                   10
ctg gca ggc agc gcc act ttg gtc gcc ctt ggg gca ctg gcc ttg tac 6100
Leu Ala Gly Ser Ala Thr Leu Val Ala Leu Gly Ala Leu Ala Leu Tyr
                15                  20                  25
gtc gcg aag ccc tcc ggc tac ggg aag cac acg gag agc ctg aag ccg 6148
Val Ala Lys Pro Ser Gly Tyr Gly Lys His Thr Glu Ser Leu Lys Pro
            30                  35                  40
gcg gct acc cgc ctg cca gcc cgc gcc gcc tgg ttc ctg cag gag ctg 6196
Ala Ala Thr Arg Leu Pro Ala Arg Ala Ala Trp Phe Leu Gln Glu Leu
        45                  50                  55
cct tcc ttc gcg gtg ccc gcg ggg atc c 6224
Pro Ser Phe Ala Val Pro Ala Gly Ile
    60                  65

<210> 2
<211> 20
<212> DNA
<213> Artificial Sequence

<220>
<223> Synthesized with reference to a sequence in the neighborhood of the
transcription starting point of human type II 5α-reductase

<400> 2
gaaaccgctg aggaattagg     20

<210> 3
<211> 20
<212> DNA
<213> Artificial Sequence

<220>
<223> Synthesized with reference to a sequence in the neighborhood of the
transcription starting point of human type II 5α-reductase

<400> 3
   ttcgcagcaa tacccctttc     20

<210> 4
<211> 517
<212> DNA
<213> Homo sapiens

<400> 4
gaaaccgctg aggaattagg gccgggagag actggtacct gccgggggcg tgtggtgggg     60
cagagctggc actgatgctg agagtggcta aggagcgcgg cgccccagag cagaagggct     120
ggcagacgct cagagagcca ggatggttca gggtccaagg aaggtcctat gttgggtggg     180
agctgtgagg gagtgaaagt gcatgaggaa ccggaggaga tggaaagacc ttggcttggg     240
tgttcgaggg tgggactgcg tggtgaccga cggcacagag ggtgtgtgtt ggggcggaag     300
aaccacccca gctgaatcgt ccccgtgggg ttttcttccc gtgtcttagt tccagaagtt     360
gccgcatcag acgctaatag ttgaggaaca agtcatggaa ggacagccta agcgggaggt     420
gaatgtaaag ccgtggagag ggcgggcgaa ctaagaaggc cttcgttctc ctccggccac     480
cgcggctgca tccttgagaa aggggtattg ctgcgaa     517

<210> 5
<211> 35
<212> DNA
<213> Artificial Sequence

<220>
<223> Synthesized with reference to a sequence in the neighborhood of the
transcription starting point of human type II 5α-reductase

<400> 5
ccaccgcggc tgcatccttg agaaaggggt attgc     35

<210> 6
<211> 35
<212> DNA
<213> Artificial Sequence

<220>
<223> Synthesized with reference to a sequence in the neighborhood of the
transcription starting point of human type II 5α-reductase

<400> 6
tttccgcggc gcgccgtgtt cctcgccggt ggccg     35

<210> 7
<211> 30
<212> DNA
<213> Artificial Sequence

<220>
<223> Synthesized with reference to the cDNA base sequence of human
SRY (in the neighborhood of the transcription starting point)

<400> 7

ggaattctat gcaatcatat gcttctgcta 30

<210> 8
<211> 30
<212> DNA
<213> Artificial Sequence

<220>
<223> Synthesized with reference to the cDNA base sequence of human
SRY (in the neighborhood of the stop codon)

<400> 8
agaattcagc tttgtccagt ggctgtagcg 30